(19) Europäisches Patentamt
European Patent Office
Office européen des brevets

(11) **EP 4 342 848 A1**

(12) **EUROPEAN PATENT APPLICATION**
published in accordance with Art. 153(4) EPC

(43) Date of publication:
**27.03.2024 Bulletin 2024/13**

(21) Application number: **22804652.0**

(22) Date of filing: **16.05.2022**

(51) International Patent Classification (IPC):
*C01G 23/053* (2006.01)     *C09C 1/36* (2006.01)
*C09C 3/06* (2006.01)       *C09C 3/08* (2006.01)
*A61Q 17/04* (2006.01)      *C09D 201/00* (2006.01)
*C09D 11/00* (2014.01)      *A61K 8/29* (2006.01)
*C09D 7/62* (2018.01)

(52) Cooperative Patent Classification (CPC):
**A61K 8/29; A61Q 17/04; C01G 23/053; C09C 1/36; C09C 3/06; C09C 3/08; C09D 7/62; C09D 11/00; C09D 201/00**

(86) International application number:
**PCT/JP2022/020415**

(87) International publication number:
**WO 2022/244741 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.05.2021 JP 2021083606**

(71) Applicant: **Tayca Corporation**
**Osaka-shi,**
**Osaka 551-0022 (JP)**

(72) Inventors:
• **EJIRI, Kazumasa**
**Osaka-shi, Osaka 551-0022 (JP)**
• **SHIBATA, Kazuya**
**Osaka-shi, Osaka 551-0022 (JP)**

(74) Representative: **Müller-Boré & Partner**
**Patentanwälte PartG mbB**
**Friedenheimer Brücke 21**
**80639 München (DE)**

(54) **TITANIUM OXIDE POWDER AND METHOD FOR PRODUCING SAME**

(57)     In a titanium oxide powder of this invention, a ratio ($I_A/I_R$) of a peak intensity ($I_A$) of anatase-type crystals to a peak intensity ($I_R$) of rutile-type crystals as measured by X-ray diffractometry is 0.1 or less; particles contained in the powder have an average short-axis length from 10 to 50 nm and an average aspect ratio from 1 to 3; a ratio ($A_{450}/A_{320}$) of an absorbance $A_{450}$ at a wavelength of 450 nm to an absorbance $A_{320}$ at a wavelength of 320 nm is from 0.015 to 0.5; and the powder contains a nitrogen atom and a peak derived from the nitrogen atom is observed from 395 to 402 eV in ESCA (Electron Spectroscopy for Chemical Analysis) measurement. A titanium oxide powder is thus provided that contains rutile-type crystals as a main component and has an excellent UVA shielding effect, high safety for the human body, and good color tone.

[FIG. 1]

EP 4 342 848 A1

**Description**

TECHNICAL FIELD

The present invention relates to a titanium oxide powder, use of the same, and a method for producing the same.

BACKGROUND ART

Sunscreen cosmetics are widely used because sunburn caused by ultraviolet rays has an adverse effect on the skin. Moreover, not only sunscreen cosmetics but also makeup cosmetics are often required to have an ultraviolet shielding effect. For the purpose of that, cosmetics containing inorganic particles such as titanium oxide and zinc oxide and/or organic ultraviolet absorbers are developed. Among these, titanium oxide is widely used because it has a high ultraviolet shielding effect and is less likely to cause skin troubles caused by an organic ultraviolet absorber. In particular, titanium oxide fine particles having a particle size of several tens of nm or less are excellent in visible light transmission because their size is smaller than the wavelength of light, and in cosmetics containing such particles, whitishness derived from titanium oxide is reduced and transparency during use is excellent (see Patent Documents 1 through 3).

However, the finer and more dispersed it is for improving transparency, the more susceptible to Rayleigh scattering it is. Intensity of Rayleigh scattering is stronger as the particles become smaller, and blue light is more likely to be scattered than red light, so that a bluish color is felt when a cosmetic containing fine particles of titanium oxide is applied to the skin. The pale color is undesirable as a cosmetic because the skin color looks unhealthy.

To address the problem, there is an approach that a small amount of iron oxide ($Fe_2O_3$) is added to cancel the bluish color. However, since iron oxide shows not only yellow, which is the complementary color of blue, but also red, it is inevitable that the cosmetic becomes dull in hue. Thus, a better approach is desired.

Patent Document 4 describes a titanium oxide powder containing rutile-type crystals doped with bivalent sulfur atoms ($S^{2-}$) to obtain a cosmetic that cancels the bluish color derived from Rayleigh scattering and has good transparency and good color tone. Patent Document 4 also describes that particles contained in the titanium oxide powder have an average short-axis length from 4 to 13 nm and an average aspect ratio from 2 to 7. Thus, a smaller particle diameter improves the transparency and also improves the UVB (from 280 to 320 nm) shielding effect while reduces the UVA (from 320 to 400 nm) shielding effect. There is a demand for improvement in the UVA shielding effect to keep the fair skin by effectively inhibiting deposition of melanin pigments due to sunburn.

It is known that fine particles of titanium oxide with a larger particle diameter have an improved UVA shielding effect. However, a larger particle diameter causes a problem of whitishness and reduction of the transparency. Meanwhile, rutile-type titanium oxide particles in a spindle shape widely used as titanium oxide for the use of ultraviolet shielding may have an increased particle diameter by proceeding with crystal growth, whereas the particles turn out to have a large aspect ratio (long-axis length / short-axis length) because the crystals grow mainly in a long-axis direction during the crystal growth. In recent years, fine particles with a large aspect ratio are considered to cause a problem of safety for the human body for the reasons such as strong irritation to the skin, and for example, the EU Cosmetics Regulation requires the titanium oxide fine particles to have an aspect ratio of 4.5 or less. Accordingly, there is a problem with the approach of increasing the particle diameter of titanium oxide simply by crystal growth.

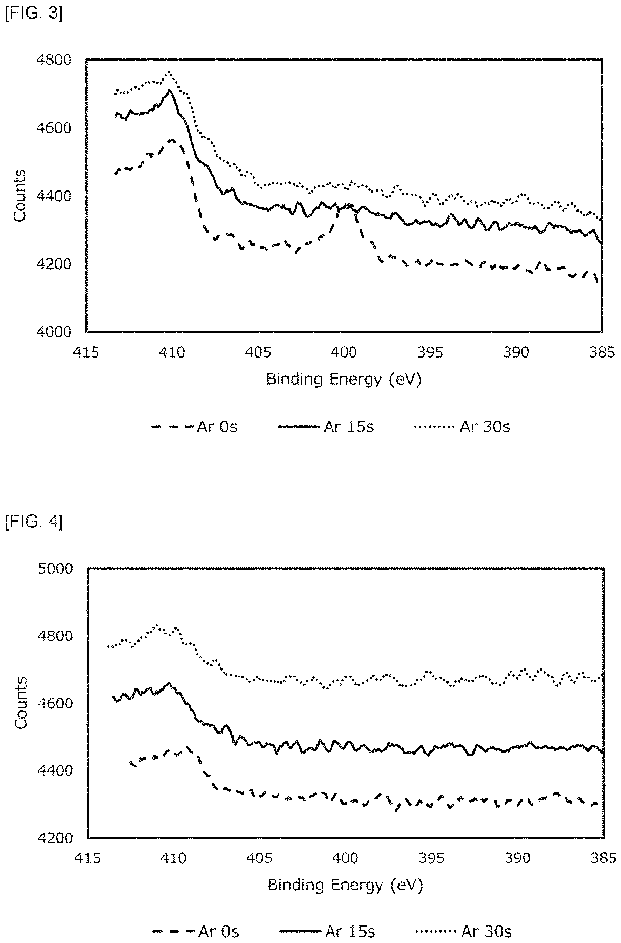

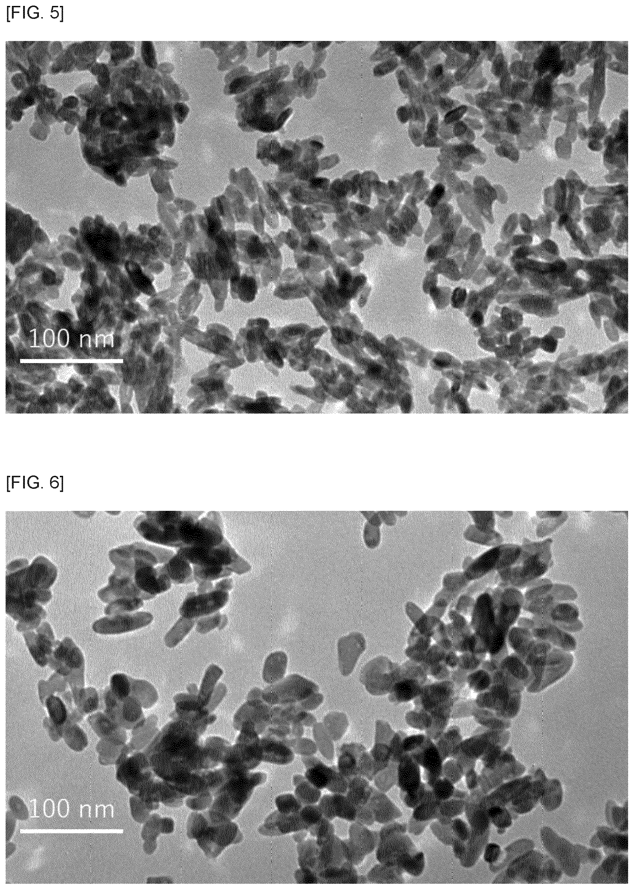

Non-Patent Document 1 describes a photocatalyst of titanium oxide nanoparticles doped with nitrogen and containing both anatase-type crystals and rutile-type crystals. It is described that the titanium oxide nanoparticles absorb light from 400 to 500 nm (Fig. 4b). In a specific example, there is a description of examples where the ratio of the rutile-type crystals was 18.3%, 20.2%, and 36.9% and the photocatalytic activity was best at 20.2% (refer to Table 1 and Fig. 5). That is, Non-Patent Document 1 describes that the photocatalytic activity was high when the anatase-type crystals were contained much more than the rutile-type crystals.

PRIOR ART DOCUMENT

PATENT DOCUMENTS

Patent Document 1: JP 2010-173863 A
Patent Document 2: JP 2011-001199 A
Patent Document 3: JP 2014-084251A
Patent Document 4: WO 2020/230812

NON-PATENT DOCUMENTS

Non-patent Document 1: J. Liu et al., Catalysts, 2020, 10, 1126

SUMMARY OF THE INVENTION

PROBLEMS TO BE SOLVED BY THE INVENTION

The present invention has been made to solve the above problems, and an object thereof is to provide a titanium oxide powder containing rutile-type crystals as a main component and having an excellent UVA shielding effect, high safety for the human body, and good color tone. Another object thereof is to provide a dispersion, particularly a cosmetic, having good transparency and good color tone. Still another object thereof is to provide a preferred method for producing such a titanium oxide powder.

MEANS FOR SOLVING THE PROBLEMS

The above problems are solved by providing a titanium oxide powder, wherein a ratio ($I_A/I_R$) of a peak intensity ($I_A$) of anatase-type crystals to a peak intensity ($I_R$) of rutile-type crystals as measured by X-ray diffractometry is 0.1 or less, particles contained in the powder have an average short-axis length from 10 to 50 nm and an average aspect ratio from 1 to 3, a ratio ($A_{450}/A_{320}$) of an absorbance $A_{450}$ at a wavelength of 450 nm to an absorbance $A_{320}$ at a wavelength of 320 nm is from 0.015 to 0.5, and the powder contains a nitrogen atom and a peak derived from the nitrogen atom is observed from 395 to 402 eV in ESCA (Electron Spectroscopy for Chemical Analysis) measurement.

It is herein preferred that no peaks of anatase-type crystals are observed in the X-ray diffractometry. It is also preferred that the powder has a specific surface area from 25 to 100 $m^2/g$. It is also preferred that an absorbance $A_{600}$ at a wavelength of 600 nm is 0.1 or less. It is also preferred that, in an L*a*b* color scale, an L* value is from 92 to 99, an a* value is from -5 to 2, and a b* value is from 3 to 30.

In a preferred embodiment, the surface of the particles contained in the powder is coated with an inorganic compound and/or organic compound layer. It is herein preferred that the surface of the particles is coated with an inorganic compound layer and the inorganic compound contains at least one element selected from the group consisting of aluminum, magnesium, calcium, silicon, zinc, titanium, zirconium, iron, cerium, and tin. It is also herein preferred that the surface of the particles is coated with an organic compound layer and the organic compound is at least one selected from the group consisting of a fatty acid or a salt thereof, a silicone compound, a coupling agent, and a fluorine compound. It is also herein preferred that, in an L*a*b* color scale, an L* value is from 92 to 99, an a* value is from -5 to 2, and a b* value is from 2 to 30.

A preferred embodiment is a dispersion comprising the above titanium oxide powder dispersed in a dispersion medium. Another preferred embodiment is a cosmetic, a paint, or an ink comprising the above titanium oxide powder. Still another preferred embodiment is a toner comprising, as an external additive, the above titanium oxide powder.

The above problems are also solved by providing a method for producing a titanium oxide powder, comprising:

an alkalization step of adding an alkali metal hydroxide to an aqueous dispersion of hydrous titanium oxide to provide an alkali metal titanate;
an acidification step of adding hydrochloric acid to the aqueous dispersion of the alkali metal titanate to provide titanium oxide containing rutile-type crystals;
an impregnation step of impregnating the titanium oxide with a nitrogenous compound; and
a calcination step of calcining from 200 to 600°C.

It is herein preferred that the titanium oxide before calcining has a specific surface area from 120 to 300 $m^2/g$. It is also preferred that, in the impregnation step, the hydrochloric acid remained after the acidification step is neutralized by a basic nitrogenous compound and the titanium oxide is impregnated with the nitrogenous compound.

EFFECTS OF THE INVENTION

The titanium oxide powder of the present invention contains rutile-type crystals as a main component and has an excellent UVA shielding effect, high safety for the human body, and good color tone. This allows a dispersion, particularly a cosmetic, to be provided that can cancel the bluish color derived from Rayleigh scattering and has good transparency and good color tone. In addition, the production method of the present invention allows convenient production of such a titanium oxide powder.

BRIEF DESCRIPTION OF DRAWINGS

Fig. 1 is an X-ray diffractometry chart for a titanium oxide powder (after calcination) with a specific surface area of approximately 80 $m^2$/g produced in Example 1.

Fig. 2 is an ESCA spectrometry chart for the titanium oxide powder (after calcination) with a specific surface area of approximately 80 $m^2$/g produced in Example 1.

Fig. 3 is an ESCA spectrometry chart for a titanium oxide powder (after calcination) with a specific surface area of approximately 60 $m^2$/g produced in Example 1.

Fig. 4 is an ESCA spectrometry chart for a titanium oxide powder "MT-100Z" used in Comparative Example 3.

Fig. 5 is a transmission electron microscope (TEM) image for the titanium oxide powder (after calcination) with a specific surface area of approximately 80 $m^2$/g produced in Example 1.

Fig. 6 is a transmission electron microscope (TEM) image for the titanium oxide powder (after calcination) with a specific surface area of approximately 60 $m^2$/g produced in Example 1.

Fig. 7 is a graph comparing the absorbance of the titanium oxide powder (after calcination) with a specific surface area of approximately 80 $m^2$/g produced in Example 1 with that produced in Comparative Example 2.

Fig. 8 is a graph comparing the light transmittance of a coating film of an emulsified preparation with a concentration of 10 mass% produced in Example 1 with that produced in Comparative Example 3.

MODES FOR CARRYING OUT THE INVENTION

In a titanium oxide powder of the present invention, a ratio ($I_A$/$I_R$) of a peak intensity ($I_A$) of anatase-type crystals to a peak intensity ($I_R$) of rutile-type crystals as measured by X-ray diffractometry is 0.1 or less,

particles contained in the powder have an average short-axis length from 10 to 50 nm and an average aspect ratio from 1 to 3,

a ratio ($A_{450}$/$A_{320}$) of an absorbance $A_{450}$ at a wavelength of 450 nm to an absorbance $A_{320}$ at a wavelength of 320 nm is from 0.015 to 0.5, and

the powder contains a nitrogen atom and a peak derived from the nitrogen atom is observed from 395 to 402 eV in ESCA (Electron Spectroscopy for Chemical Analysis) measurement. Such a titanium oxide powder was produced for the first time by intense investigation by the present inventors. A detailed description is given below.

In the titanium oxide powder of the present invention, the ratio ($I_A$/$I_R$) of a peak intensity ($I_A$) of anatase-type crystals to a peak intensity ($I_R$) of rutile-type crystals as measured by X-ray diffractometry is 0.1 or less. A higher content of anatase-type crystals causes higher photocatalytic activity, and thus when the powder is used for application of being exposed to ultraviolet light or visible light, an organic substance in contact with the titanium oxide particles is likely to be degraded. In particular, it is not desirable to add the titanium oxide to a cosmetic because it easily irritates the skin, leading to troubles.

In this context, the peak intensity ($I_R$) of the rutile-type crystal is a peak intensity ($I_R$) of the rutile-type titanium oxide (110 plane) around $2\theta = 27.5°$ in X-ray diffractometry. In addition, the peak intensity ($I_A$) of the anatase-type crystal is a peak intensity ($I_A$) of the anatase-type titanium oxide (101 plane) around $2\theta = 25.3°$. These peak intensities can be obtained using an X-ray diffractometer described in Examples herein or a device equivalent thereto under measurement conditions described in Examples herein or measuring conditions equivalent thereto. Each peak appearing in the X-ray diffractometry charts does not have to be an independent peak, and an anatase-type crystal peak may be observed as a shoulder peak of a rutile-type crystal peak. The peaks are separated by analysis software attached to the device, and the intensity of each peak is calculated.

The ratio ($I_A$/$I_R$) is preferably 0.05 or less, and more preferably no peaks of anatase-type crystals are observed in the X-ray diffractometry. In this context, "no peaks of anatase-type crystals are observed" means that no peaks of the anatase-type crystals are detected in measurement under the conditions described in Examples herein. Under the conditions of Examples herein, the ratio ($I_A$/$I_R$) is less than 0.03.

The particles contained in the titanium oxide powder of the present invention have an average short-axis length from 10 to 50 nm. The average short-axis length of 10 nm or more improves the UVA (320 - 400 nm) shielding effect. An average short-axis length of less than 10 nm improves the UVB (280 - 320 nm) shielding effect while reducing the UVA shielding effect. Although it is important to shield UVB with a risk of causing inflammation in the skin, it is also important to shield UVA causing the skin to be tanned by deposition of melanin pigments, and particularly from an aesthetic perspective, the UVA shielding is very important. The average short-axis length is preferably 12 nm or more and more preferably 14 nm or more. Meanwhile, the average short-axis length of 50 nm or less improves the transparency of the coating film containing the titanium oxide. The average short-axis length is preferably 40 nm or less and more preferably 30 nm or less. The average short-axis length and average long-axis length are obtained by taking a transmission electron microscope (TEM) image and performing image processing of the particles.

The particles contained in the titanium oxide powder of the present invention have an average aspect ratio (average long-axis length / average short-axis length) from 1 to 3. In general, acicular inorganic particles have a risk of irritating the lung and skin and thus there is a concern for safety for the human body. The average aspect ratio of 3 or less improves the safety for the human body. In addition, having such a small average aspect ratio substantially reduces the unsmooth feeling typical of powder when a cosmetic containing titanium oxide is applied to the skin and thus gives an excellent feeling of use. The average aspect ratio is more preferably 2.5 or less and even more preferably 2 or less.

In the titanium oxide powder of the present invention, the ratio ($A_{450}/A_{320}$) of the absorbance $A_{450}$ at a wavelength of 450 nm to the absorbance $A_{320}$ at a wavelength of 320 nm is from 0.015 to 0.5. The ratio ($A_{450}/A_{320}$) of 0.015 or more allows blue light to be absorbed and thus the blue color derived from Rayleigh scattering to be effectively cancelled. Moreover, such a powder has a color tone similar to the skin color and thus the transparency when applied to the skin is improved. The ratio ($A_{450}/A_{320}$) is preferably 0.025 or more. Meanwhile, a ratio ($A_{450}/A_{320}$) of more than 0.5 causes marked coloration and thus it is difficult to be used for cosmetics. The ratio ($A_{450}/A_{320}$) is preferably 0.4 or less and more preferably 0.3 or less. The absorbance of the titanium oxide powder is obtained by measurement by diffuse reflectance spectroscopy using a spectrophotometer and a sample of a shaped article produced by compressing the powder.

The titanium oxide powder of the present invention contains a nitrogen atom and a peak derived from the nitrogen atom is observed from 395 to 402 eV in ESCA (Electron Spectroscopy for Chemical Analysis) measurement. In the ESCA measurement, a peak of a binding energy of Ti-N is considered to appear around 397 eV and a peak of a binding energy of Ti-N-O and Ti-O-N is considered to appear around 400 eV. The presence of these peaks is determined by whether a peak is observed in the ESCA measurement under the conditions described in Examples herein. If any of these peaks is observed, the powder is determined to contain a nitrogen atom. It should be noted that peaks derived from a scandium atom are known to appear at 406 eV and 401.7 eV as doublet peaks (width 4.3 eV) in the ESCA measurement. Among them, the peak at 401.7 eV overlaps the range from 395 to 402 eV defined in the present invention while this peak is not taken into account. In addition, in the titanium oxide powder of the present invention, no scandium atoms are contained in general.

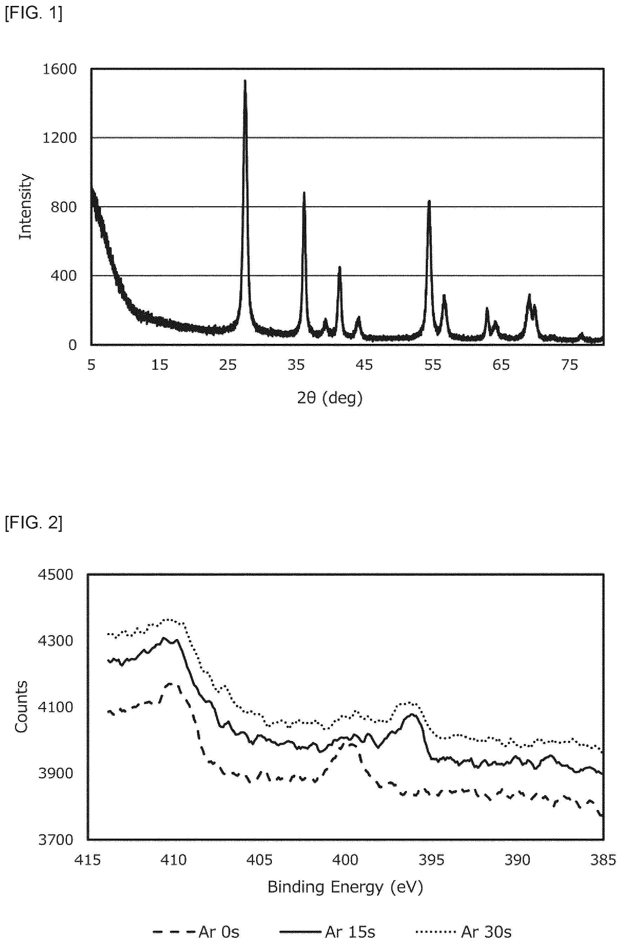

As illustrated in Fig. 2, in a titanium oxide powder with a specific surface area of approximately 80 m$^2$/g obtained by neutralization by ammonia and then calcination at 380°C in Example 1, a peak around 400 eV is observed on the surface of the particles and a peak around 397 eV is observed inside the particles. Meanwhile, as illustrated in Fig. 3, in a titanium oxide powder with a specific surface area of approximately 60 m$^2$/g obtained by neutralization by ammonia and then calcination at 480°C in Example 1, a peak around 400 eV is observed on the surface of the particles while no peaks around 397 eV are observed inside the particles. For this reason, if the particles are enlarged by calcination at higher temperatures, there is a possibility that the Ti-N bonds once formed inside the particles disappear. In contrast, as illustrated in Fig. 4, in a commercially available titanium oxide powder that is neither neutralized by ammonia nor calcined used in Comparative Example 3, no peaks are observed in the range from 395 to 402 eV. It should be noted that, in the case of titanium oxide particles obtained by calcination without being neutralized by ammonia as in Comparative Example 2, a peak around 400 eV is observed on the surface of the particles similar to the case of the titanium oxide powder with a specific surface area of approximately 60 m$^2$/g in Example 1 while no peaks are observed around 397 eV inside the particles. The peak around 400 eV is considered to be developed by, during calcination in an atmosphere, binding nitrogen atoms derived from the nitrogen molecules in the atmosphere to the surface of the titanium oxide particles. However, different from the titanium oxide powders in Examples 1 through 4, the titanium oxide powder in Comparative Example 2 is not capable of sufficiently absorbing the light at 450 nm and has a ratio ($A_{450}/A_{320}$) of the absorbance $A_{450}$ at a wavelength of 450 nm to the absorbance $A_{320}$ at a wavelength of 320 nm of 0.015 or less. Because of the reasons as above, it is estimated that neutralization by ammonia followed by calcination at high temperatures causes some structure that absorbs the light at 450 nm to remain even if the Ti-N bonds once formed inside the particles disappear. Accordingly, the peak derived from the nitrogen atoms is observed from 395 to 402 eV and the ratio ($A_{450}/A_{320}$) is from 0.015 to 0.5, thereby allowing production of a titanium oxide powder with good color tone.

It is preferred that, in the titanium oxide powder of the present invention, an absorbance $A_{600}$ at a wavelength of 600 nm is 0.1 or less. The absorbance $A_{600}$ of 0.1 or less allows inhibition of red light absorption. If red light is also absorbed in addition to the blue light, the color tone becomes dull and it is undesirable to be used as a cosmetic. The absorbance $A_{600}$ is more preferably 0.07 or less and even more preferably 0.05 or less.

It is preferred that, in the titanium oxide powder of the present invention, in an L*a*b* color scale, an L* value is from 92 to 99, an a* value is from -5 to 2, and a b* value is from 3 to 30. The L* value of 92 or more allows production of a powder exhibiting high whiteness without dullness. The L* value is more preferably 93 or more and even more preferably 95 or more. The a* value of 2 or less causes inhibition of redness. The a* value is more preferably 0 or less and even more preferably -1 or less. Moreover, the b* value of 3 or more allows effective cancellation of bluish tint. The b* value is more preferably 4 or more and even more preferably 5 or more. Meanwhile, a b* value of more than 30 sometimes causes yellow color to be too deep. The b* value is more preferably 20 or less and even more preferably 15 or less.

The titanium oxide powder of the present invention preferably has an iron content of 300 ppm or less. The iron content (ppm) is a mass of iron element based on a mass of the powder. The iron content of 300 ppm or less allows production of a dispersion, particularly a cosmetic, exhibiting excellent transparency without dullness. The iron content is more preferably 200 ppm or less and even more preferably 150 ppm or less. A too high iron content causes a risk of aggregating the titanium oxide particles and degrading the transparency. It should be noted that all titanium oxide powders produced in Examples and Comparative Examples particularly contain no iron elements.

It is preferred that the titanium oxide powder of the present invention has a specific surface area from 25 to 100 $m^2$/g. The specific surface area of 25 $m^2$/g or more allows production of a dispersion, particularly a cosmetic, exhibiting excellent transparency with reduced whitishness. The specific surface area is more preferably 40 $m^2$/g or more and even more preferably 50 $m^2$/g or more. Meanwhile, the specific surface area of 100 $m^2$/g or less allows production of a dispersion, particularly a cosmetic, capable of effectively shielding UVA and inhibiting formation of melanin pigments due to sunburn. The specific surface area is more preferably 90 $m^2$/g or less.

A description is given below to a method for producing the titanium oxide powder of the present invention. The production method preferably has: an alkalization step of adding an alkali metal hydroxide to an aqueous dispersion of hydrous titanium oxide ($TiO_2 \cdot nH_2O$) to provide an alkali metal titanate; an acidification step of adding hydrochloric acid to the aqueous dispersion of the alkali metal titanate to provide titanium oxide ($TiO_2$) containing rutile-type crystals; an impregnation step of impregnating the titanium oxide with a nitrogenous compound; and a calcination step of calcining from 200 to 600°C.

In the alkalization step, an alkali metal hydroxide is added to an aqueous dispersion of hydrous titanium oxide (titanium dioxide hydrate: $TiO_2 \cdot nH_2O$) to provide an alkali metal titanate. Examples of a method for producing the hydrous titanium oxide used in this procedure include, but not particularly limited to, a production method comprising heating an aqueous solution of titanyl sulfate ($TiOSO_4$) for hydrolysis. The hydrous titanium oxide thus obtained usually contains anatase-type crystals.

Examples of the alkali metal hydroxide to be added to the aqueous dispersion of hydrous titanium oxide include sodium hydroxide, potassium hydroxide, and lithium hydroxide, preferably sodium hydroxide and potassium hydroxide, and particularly preferably sodium hydroxide. The mole number of the alkali metal hydroxide added in this procedure is preferably from 2 to 20 times the mole number of titanium element in the hydrous titanium oxide. A heating temperature in this procedure is preferably from 60°C to 120°C. Thus, an aqueous dispersion of alkali metal titanate is obtained. Examples of the alkali metal titanate include sodium titanate ($Na_2O_7Ti_3$), potassium titanate, and lithium titanate.

In the acidification step after the alkalization step, hydrochloric acid is added to the aqueous dispersion of the alkali metal titanate to give titanium oxide ($TiO_2$) containing rutile-type crystals. Addition of hydrochloric acid to acidify the aqueous dispersion allows production of an aqueous dispersion in which titanium oxide particles containing rutile-type crystals are dispersed. The amount of hydrochloric acid to be added is an amount sufficient to neutralize the excess alkali in the aqueous dispersion and to acidify the aqueous dispersion. It is preferred to add hydrochloric acid followed by heating for aging, and the conditions are adjusted to allow particle growth. A preferred aging temperature is from 40°C to 110°C. The aging temperature is more preferably 60°C or more. The aging temperature is also more preferably 105°C or less. A preferred aging time is from 2 minutes to 24 hours. The aging time is more preferably 5 minutes or more. The aging time is also more preferably 10 hours or less.

In the impregnation step after the acidification step, the titanium oxide thus obtained is impregnated with a nitrogenous compound. The nitrogenous compound may be added to an aqueous dispersion of the titanium oxide produced in the acidification step for impregnation followed by drying, or the aqueous dispersion may be dried followed by being impregnated with the nitrogenous compound. The nitrogenous compound used in this procedure may be, but not particularly limited to, ammonia, ammonium bicarbonate, amine, urea, and the like.

It is preferred that, in the impregnation step, the hydrochloric acid remained after the acidification step is neutralized by a basic nitrogenous compound and the titanium oxide is impregnated with the nitrogenous compound. It is thus possible to uniformly impregnate the nitrogenous compound in the aqueous dispersion containing the titanium oxide particles simultaneously with the neutralization operation. Examples of the basic nitrogenous compound used in this procedure include ammonia, ammonium bicarbonate, amine, and the like. It is particularly preferred to add aqueous ammonia because of the convenient and inexpensive operation. The titanium oxide particles are heated after the neutralization operation to remove water for drying. A preferred drying temperature is 70°C or more and less than 200°C. After drying, pulverization and sieving may be carried out as needed to produce a titanium oxide powder (before calcination).

In addition, the hydrochloric acid remained after the acidification step may be neutralized by a base other than the nitrogenous compound. Examples of such a base include sodium hydroxide, potassium hydroxide, and the like. The titanium oxide particles are heated after the neutralization operation to remove water for drying. A preferred drying temperature is 70°C or more and less than 200°C. After drying, pulverization and sieving may be carried out as needed to produce a titanium oxide powder (before calcination). The titanium oxide powder (before calcination) may be impregnated with a solution of the basic nitrogenous compound, preferably an aqueous solution thereof, to be used in the calcination step later.

In the calcination step, the titanium oxide powder is calcined from 200°C to 600°C. This allows the titanium oxide particles to have an increased short-axis length and a reduced aspect ratio. The calcination temperature is preferably 250°C or more and more preferably 300°C or more. The calcination temperature is also preferably 550°C or less and more preferably 500°C. The atmosphere for the calcination may be, but not particularly limited to, an air atmosphere. In this case, nitrogen atoms derived from the nitrogen molecules in the atmosphere sometimes bind to the surface of the titanium oxide particles. After calcination, pulverization and sieving may be carried out as needed to produce a titanium oxide powder (after calcination).

The titanium oxide powder of the present invention may be directly used for various applications while the surface is preferably coated. That is, a preferred embodiment of the present invention is a titanium oxide powder wherein the surface of titanium oxide particles contained in the above powder is coated with an inorganic compound and/or organic compound layer.

The inorganic compound with which the titanium oxide particles are coated preferably contains at least one element selected from the group consisting of aluminum, magnesium, calcium, silicon, zinc, titanium, zirconium, iron, cerium and tin. Coating with the compound containing these elements allows an improvement in durability and dispersion stability of the titanium oxide particles. An aluminum compound is particularly preferred, and coating in the form of aluminum hydroxide is preferable, which allows an improvement in dispersion stability and inhibition of photocatalytic activity typical of titanium oxide. Examples of a method for coating with aluminum hydroxide include a method comprising adding a salt, such as aluminum chloride, to a slurry containing titanium oxide particles and hydrolyzing the slurry to precipitate aluminum hydroxide on the surface of the titanium oxide particles. A preferable content of aluminum in the titanium oxide powder is from 2 to 30 parts by mass in terms of $Al_2O_3$ based on 100 parts by mass of $TiO_2$.

Examples of the organic compound with which the titanium oxide particles are coated include at least one selected from the group consisting of a fatty acid or a salt thereof, a silicone compound, a coupling agent, and a fluorine compound. Among them, a fatty acid or a salt thereof is preferred, which can impart lipophilicity to the surface of the titanium oxide particles and facilitates dispersion in an oil phase. In particular, when being used as a cosmetic and the like, the cosmetic applied to the skin is less likely to come off due to sweat or rain, and its durability is improved. The fatty acid used here is preferably a higher fatty acid having 12 to 30 carbon atoms, and the salt thereof is preferably an aluminum salt. Examples of a method for coating with the fatty acid or a salt thereof include a method comprising adding a fatty acid salt of an alkali metal to a slurry containing titanium oxide particles, followed by adding a strong acid, such as sulfuric acid, to precipitate the free fatty acid on the surface of the titanium oxide particles. A preferable content of the fatty acid or a salt thereof in the titanium oxide powder is from 2 to 50 parts by mass based on 100 parts by mass of $TiO_2$. The content is the amount converted to the free fatty acid.

The layer of the inorganic compound coating the surface of the titanium oxide particles does not have to be a uniform layer and may partially cover the surface. The same applies to the layer of the organic compound. The layer of the inorganic compound and the layer of the organic compound may be formed as separate layers or one layer may contain both the inorganic compound and the organic compound. In a preferred embodiment, the surface of the titanium oxide particles is covered with a layer containing aluminum hydroxide and a higher fatty acid having 12 to 30 carbon atoms or an aluminum salt thereof.

In the case of the titanium oxide powder having the surface of the titanium oxide particles coated with the inorganic compound and/or organic compound layer, it is preferred that, in the L*a*b* color scale, the L* value is from 92 to 99, the a* value is from -5 to 2, and the b* value is from 2 to 30. Because the surface coating causes a reduction in the b* value compared with that with no coating, the preferred lower limit of the b* value for the coated titanium oxide powder is lowered to 2. More preferred ranges for the L* value, the a* value, and the b* value are the same as those of the titanium oxide powder with no coating.

A dispersion prepared by dispersing the titanium oxide powder of the present invention thus obtained in a dispersion medium is a preferred embodiment. Here, the dispersion medium may be water or an organic solvent, or alternatively a mixed solvent of water and an organic solvent or an emulsion formed from water and an organic solvent.

Preferred uses are cosmetics, paints, inks, toners, and so on containing the titanium oxide powder of the present invention.

Among them, a particularly suitable use is a cosmetic, which is preferably used as a cosmetic having an ultraviolet shielding effect, in particular a cosmetic capable of inhibiting deposition of melanin pigments. The cosmetic of the present invention is allowed to contain inorganic pigments and/or organic pigments other than the titanium oxide powder of the present invention. Examples of the inorganic pigments allowed to use include titanium oxide, zinc oxide, red iron oxide, yellow iron oxide, black iron oxide, ultramarine, Prussian blue, cerium oxide, talc, white mica, synthetic mica, phlogopite, black mica, synthetic fluorine phlogopite, mica titanium, micaceous iron oxide, sericite, zeolite, kaolin, bentonite, clay, silicic acid, silicic anhydride, magnesium silicate, aluminum silicate, calcium silicate, barium sulfate, magnesium sulfate, calcium sulfate, calcium carbonate, magnesium carbonate, boron nitride, bismuth oxychloride, alumina, zirconium oxide, magnesium oxide, chromium oxide, calamine, carbon black, hydroxyapatite, composites thereof, and the like. Examples of the organic pigments allowed to use include a silicone powder, a polyurethane powder, a cellulose powder, a nylon powder, a silk powder, a polymethyl methacrylate (PMMA) powder, starch, a polyethylene powder, a polystyrene powder, a tar dye, a natural dye, composites thereof, and the like.

A cosmetic of the present invention may contain other ingredients depending on the purpose. For example, a pH adjuster, a humectant, a thickener, a surfactant, a dispersion stabilizer, a preservative, an antioxidant, a sequestering agent, an astringent, an anti-inflammatory agent, an ultraviolet absorber, a perfume, and so on may be added as appropriate.

Examples of the form of the cosmetic of the present invention include an emulsion, a lotion, an oil, a cream, a paste, and the like. Examples of the specific uses thereof include sunscreen cosmetics; makeup cosmetics such as makeup bases, foundations, concealers, control colors, lipsticks, lip balms, eye shadows, eye liners, mascaras, cheek colors, and manicures; skin care cosmetics; and hair care cosmetics.

When the titanium oxide powder of the present invention is used for a paint or ink, the titanium oxide particles may be dispersed in a solution in which a base polymer is dissolved, or the titanium oxide particles may be dispersed in an aqueous emulsion in which base polymer particles are dispersed. In addition, additives usually added to a paint or ink may be blended, including pigments, matting agents, surfactants, dispersion stabilizers, leveling agents, thickeners, antioxidants, and ultraviolet absorbers. In addition, some paints containing fine particles of titanium oxide have so-called "flip-flop" effect that color tone varies depending on a viewing angle, and use of the titanium oxide of the present invention allows formation of a "flip-flop" coating film with less bluish color.

When the titanium oxide powder of the present invention is used as an external additive for a toner, it is used as a mixture with toner particles containing a pigment. Use of the fine particle titanium oxide allows production of a toner exhibiting excellent stability of charging performance under various environments. Here, the toner may contain various additives that are usually added.

EXAMPLES

The present invention is more specifically described with reference to Examples. The analysis methods and evaluation methods in Examples were as follows.

(1) X-ray diffractometry

A titanium oxide powder pressed flat against a sample holder with a glass plate was measured by an X-ray diffractometer (manufactured by Philips). The measurement conditions were as follows.

- Diffractometer system: XPERT-PRO
- Radiation source: $CuK\alpha$
- Scan size: $2\theta = 0.008°$
- Voltage: 45 kV
- Current: 20 mA
- Measurement range: $2\theta =$ from 5° to 100°
- Attached software for analysis: HighScore Plus

A peak intensity ($I_R$) of rutile-type titanium oxide (110 plane) around $2\theta = 27.5°$ and a peak intensity ($I_A$) of anatase-type titanium oxide (101 plane) around $2\theta = 25.3°$ were measured and their ratio ($I_A/I_R$) was calculated. In this procedure, the peak search function of the attached software was used to perform peak search under the following conditions. When the peak search is performed under these conditions, the ratio ($I_A/I_R$) at the detection limit of the peak of the anatase type crystal is 0.03. Therefore, the ratio ($I_A/I_R$) when no peaks of the anatase type crystal is detected is less than 0.03.

- Minimum significance: 0.50
- Minimum peak chip: 0.10°
- Maximum peak chip: 1.00°
- Peak base width: 2.00°
- Method: Minimum value of a secondary differential

(2) ESCA measurement

ESCA (Electron Spectroscopy for Chemical Analysis) measurement was performed using an X-ray photoelectron spectroscopic analyzer "ESCA 3400" manufactured by Shimadzu Corporation. The sample powder was compacted into a thin film and then fixed with a carbon tape for measurement. The surface was repeatedly etched by argon sputtering for 15 seconds to measure a distribution of sulfur atoms in the depth direction of the sample. It was corrected with a binding energy for C1s of 284.6 eV. The measurement and etching conditions were as follows.

(Measurement conditions)

-   X-ray source: Mg-K$\alpha$ ray
-   Filament voltage-current: 12 kV-15 mA
-   Vacuum degree: less than $1.0 \times 10^{-6}$ Pa
-   Measurement range: 385-415 eV
-   Measurement step: 0.1 eV
-   Cumulative number: 30

(Argon etching conditions)

-   Filament voltage-current: 2 kV-20 mA
-   Ion source: Argon gas
-   Vacuum degree during argon etching: $1.0 \times 10^{-4}$ Pa
-   Etching time: 15 seconds/run

(3) Shape and dimensions of particles

A long-axis length (nm) and a short-axis length (nm) of each particle were determined by taking a transmission electron microscope (TEM) image and performing image processing of the particles. An aspect ratio is a value obtained by (long-axis length / short-axis length). Measurement was performed on 200 or more particles to determine an average short-axis length (nm) and an average aspect ratio.

(4) Specific surface area

A specific surface area was measured by the BET method using a fully automatic specific surface area measuring device "Macsorb HM model-1208" manufactured by Mountech Co., Ltd. The measurement was carried out after degassing at 150°C for 20 minutes in a nitrogen gas atmosphere.

(5) Powder color

An aluminum ring for powder samples (43 $\varphi$) (manufactured by Rigaku Corporation, dimensions: outer diameter of 43 mm, inner diameter of 40 mm, height of 5 mm) was set on a medicine wrapping paper and a titanium oxide powder was filled in the ring and leveled off, and was pressure molded at 15 MPa for 30 seconds using a briquetting press for tablets ("BRE-32" manufactured by Maekawa Testing Machine Mfg. Co., Ltd.) to prepare a tablet with a thickness from 2.5 to 3.0 mm to be used as a sample for measurement. The color of the sample thus obtained was measured using a color difference meter "CR-400" manufactured by Konica Minolta Co., Ltd. After performing white calibration of the color difference meter, the L* value, the a* value, and the b* value in the L*a*b* color scale were measured.

(6) Absorbance

A tablet obtained in the same manner as that in the "(5) Powder color" above was used as a sample to obtain the absorbance by a diffuse reflectance spectroscopy with an integrating sphere spectrophotometer ("U-4100" manufactured by Hitachi High-Technologies Corp.) The measurement conditions were as follows. The measurement was performed after standard correction using a barium sulfate standard white plate.

- Scan speed: 300 nm/min
- Sampling interval: 2 nm
- Measurement wavelength: from 250 to 700 nm

An absorbance ratio ($A_{450}/A_{320}$) was calculated from the absorbance $A_{450}$ at a wavelength of 450 nm and the absorbance $A_{320}$ at a wavelength of 320 nm.

(7) Color difference of coating film

A PET film provided with a coating film partially on the surface was placed on a Bioskin (#biocolor BSC from Beaulax Co., Ltd.), which is a standard color of the skin color, to measure the color difference from the coating film side using a color difference meter ("CR-400" manufactured by Konica Minolta Co., Ltd.). After white calibration of the color difference meter, the L* value, the a* value, and the b* value in the L*a*b* color scale were measured for each of the unapplied area (blank) and the applied area on the PET film. An average value of measuring the applied area twice was used to calculate a color difference ΔE between the blank and the applied area from a mathematical expression below. A smaller ΔE value indicates less deviation from the skin color and better fit to the skin.

$$\Delta E = \sqrt{(L^*_2 - L^*_1)^2 + (a^*_2 - a^*_1)^2 + (b^*_2 - b^*_1)^2}$$

In this expression, $L^*_1$, $a^*_1$, and $b^*_1$ respectively denote the L* value, the a* value, and the b* value of the blank, and $L^*_2$, $a^*_2$, and $b^*_2$ respectively denote the L* value, the a* value, and the b* value of the applied area.

(8) Sensory evaluation of emulsified preparation (feeling of use, whiteness, bluish color, transparency)

An emulsified preparation was applied to the skin of the forearm under sunlight for evaluation by 10 panelists of the feeling of use and visual evaluation of the whiteness, the bluish color, and the transparency, varying the viewing angle. In particular, the bluish color was observed in the area where the veins were seen through blue.

Example 1

An aqueous solution of titanyl sulfate ($TiOSO_4$) was heated to 100°C and hydrolyzed to precipitate hydrous titanium oxide (titanium dioxide hydrate) ($TiO_2 \cdot nH_2O$), giving a slurry. The slurry was filtered to give a cake, which was then washed with water to obtain 35 kg (10 kg in terms of $TiO_2$) of a hydrous titanium oxide cake. The hydrous titanium oxide thus obtained contained anatase-type crystals. To this cake, 70 kg of a 48 % by mass aqueous solution of sodium hydroxide was added with stirring. The mixture was then heated and stirred at temperatures ranging from 95°C to 105°C for 2 hours to obtain a slurry of sodium titanate ($Na_2O_7Ti_3$). The slurry was filtered to prepare a cake, which was thoroughly washed with water to obtain a cake of sodium titanate. To the cake thus obtained, water was added to give a slurry containing 170 g/L of sodium titanate in terms of $TiO_2$.

To the slurry containing sodium titanate, 14.0 kg of 35 mass% hydrochloric acid was added and heated for aging at 80°C for 10 minutes. The slurry after aging was diluted with water to obtain a slurry containing 70 g/L of titanium oxide in terms of $TiO_2$. The titanium oxide thus obtained contained rutile-type crystals. This titanium oxide slurry was warmed to 80°C and adjusted to pH 7.0 with aqueous ammonia, and then aged for 30 minutes. After the aging was completed, the slurry was readjusted to pH 7.0 with aqueous ammonia or hydrochloric acid and then filtered and washed to give a titanium oxide cake. This cake was dried at 110°C, then pulverized by an impact pulverizer and then sieved through a 0.3 mm sieve to provide a titanium oxide powder (before calcination). The titanium oxide powder had an average short-axis length of 6 nm and an average aspect ratio of 3.5. The powder had a specific surface area of 204 $m^2/g$ as measured by the BET method and had the L* value of 97.99, the a* value of -0.53, and the b* value of 2.41 as measured with the color difference meter. The results of the above evaluation are summarized in Table 1.

The titanium oxide powder (before calcination) thus obtained was put in a crucible with a lid that was not airtight, and the powder was calcined at 380°C for 120 minutes to have a specific surface area of approximately 80$m^2/g$ using a box-type electric furnace ("KBF 828N1" manufactured by Koyo Thermo Systems Co., Ltd.) in an air atmosphere. The calcination thus obtained was pulverized by the impact pulverizer and sieved through a 0.3 mm sieve to obtain a titanium oxide powder (after calcination) with a specific surface area of 82.0 $m^2/g$. In addition, in a similar operation, the calcination temperature was changed to 480°C to have a specific surface area of approximately 60 $m^2/g$ to obtain a titanium oxide powder (after calcination) with a specific surface area of 60.3 $m^2/g$.

Using the titanium oxide powder (after calcination) thus obtained, analysis and evaluation were performed in accordance with the above methods. Fig. 1 illustrates an X-ray diffractometry chart for the titanium oxide powder (after calcination) with a specific surface area of approximately 80 $m^2$/g. No peaks of anatase-type crystals were detected, and the ratio ($I_A/I_R$) of the peak intensity ($I_A$) of anatase-type crystals to the peak intensity ($I_R$) of rutile-type crystals was less than 0.03. In addition, in the X-ray diffractometry of the titanium oxide powder (after calcination) with a specific surface area of approximately 60 $m^2$/g, no peaks of anatase-type crystals were detected. Moreover, in all the titanium oxide powders (after calcination) obtained in Examples 2 through 4 and Comparative Examples 1 through 2 described later, no peaks of anatase-type crystals were detected.

Fig. 2 illustrates an ESCA spectrometry chart for the titanium oxide powder (after calcination) with a specific surface area of approximately 80 $m^2$/g. In observation before etching (Ar-0 seconds), a peak was observed around a binding energy of 400 eV. In observation 15 seconds and 30 seconds after etching (Ar-15 seconds, Ar-30 seconds), a major peak was observed around a binding energy of 397 eV and a minor peak was observed around 400 eV. All the peaks were derived from the nitrogen atoms.

Fig. 3 illustrates an ESCA spectrometry chart for the titanium oxide powder (after calcination) with a specific surface area of approximately 60 $m^2$/g. In observation before etching (Ar-0 seconds), a peak derived from the nitrogen atoms was observed around a binding energy of 400 eV, whereas in observation 15 seconds and 30 seconds after etching (Ar-15 seconds, Ar-30 seconds), no peaks were observed in the binding energies ranging from 395 to 402 eV.

Fig. 5 illustrates a transmission electron microscope (TEM) image for the titanium oxide powder (after calcination) with a specific surface area of approximately 80 $m^2$/g. The average short-axis length calculated by image processing based on the TEM photograph was 12.7 nm, and the average aspect ratio was 1.60. Fig. 6 illustrates a transmission electron microscope (TEM) image for the titanium oxide powder (after calcination) with a specific surface area of approximately 60 $m^2$/g. The average short-axis length calculated by image processing based on the TEM photograph was 22.4 nm, and the average aspect ratio was 1.54.

The titanium oxide powder (after calcination) with a specific surface area of approximately 80 $m^2$/g had the L* value of 96.04, the a* value of -1.66, and the b* value of 7.93, which were measured by the color difference meter. In addition, the titanium oxide powder (after calcination) with a specific surface area of approximately 60 $m^2$/g had the L* value of 96.58, the a* value of -1.37, and the b* value of 7.58.

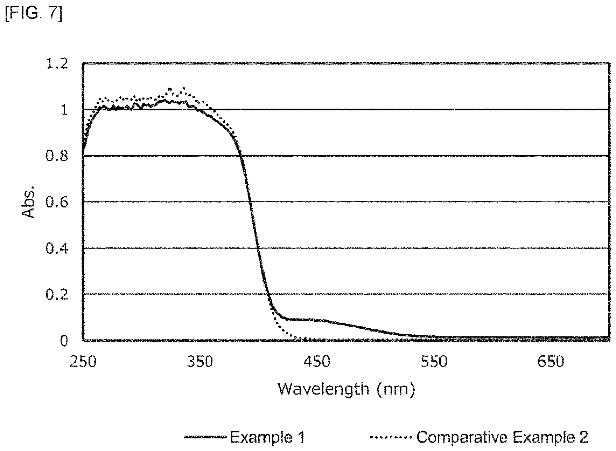

Fig. 7 illustrates a graph of the absorbance measured by the spectrophotometer of the titanium oxide powder (after calcination) with a specific surface area of approximately 80 $m^2$/g together with the absorbance of the titanium oxide powder (after calcination) obtained in Comparative Example 2 with a specific surface area of approximately 80 $m^2$/g. The titanium oxide powder of Example 1 had a characteristic absorption around 450 nm, while that of Comparative Example 2, in which the powder was not neutralized by ammonia, did not have an absorption around 450 nm.

The titanium oxide powder (after calcination) with a specific surface area of approximately 80 $m^2$/g had an absorbance at 320 nm of 1.041, an absorbance at 450 nm of 0.088, and an absorbance at 600 nm of 0.014. Accordingly, the ratio ($A_{450}/A_{320}$) was 0.085. In addition, the titanium oxide powder (after calcination) with a specific surface area of approximately 60 $m^2$/g had an absorbance at 320 nm of 1.034, an absorbance at 450 nm of 0.081, and an absorbance at 600 nm of 0.007. Accordingly, the ratio ($A_{450}/A_{320}$) was 0.078. The results of the above evaluation are summarized in Table 2 for the titanium oxide powder (after calcination) with a specific surface area of approximately 80 $m^2$/g and in Table 3 for the titanium oxide powder (after calcination) with a specific surface area of approximately 60 $m^2$/g.

Preparation of lacquer varnish

The following materials were uniformly mixed to prepare 3500 g of a lacquer varnish. The solids concentration of nitrocellulose (NC) in this procedure was 10 mass%.

- Nitrocellulose (H1/2): 500 g

(produced by Kishida Chemical Co., Ltd., solids concentration of approximately 70 mass%)

- N-butyl acetate: 1050 g
- Ethyl acetate: 700 g
- Ethylene glycol mono-n-butyl ether: 350 g
- Toluene: 900 g

Preparation of nitrocellulose paint

In a 100 mL bottle (J bottle, round shape wide mouth, manufactured by Nikko Hansen & Co., Ltd.), 40 g of the lacquer varnish thus obtained, 1.714 g of the titanium oxide powder (after calcination) with a specific surface area of

approximately 80 m$^2$/g, and 130 g of φ 0.5 mm zirconia beads (produced by Shinmaru Enterprises Corp.) as a dispersion medium were put, and dispersed in a paint conditioner ("1400-OH" produced by RED DEVIL) for 90 minutes, followed by separating the zirconia beads to prepare a paint. The paint thus obtained contained 30 mass% of titanium oxide based on the total solids.

Formation and evaluation of nitrocellulose coating film

The paint thus obtained was applied on a PET film (Lumirror 100T60 produced by Panac Co., Ltd.) using an automatic bar coater with an attached wire bar (No. 22) and dried at room temperature for 24 hours to form a coating film. The color difference ΔE of the coating film thus formed from the Bioskin was evaluated in accordance with the above method. As a result, the ΔE was 9.6. In addition, a paint and a coating film were prepared and evaluated in the same manner using the titanium oxide powder with a specific surface area of approximately 60 m$^2$/g, and the ΔE was 10.2.

Surface treatment

Water was added to the titanium oxide powder (after calcination) with a specific surface area of approximately 80 m$^2$/g or with a specific surface area of approximately 60 m$^2$/g to obtain a titanium oxide slurry, 70 g/L in terms of TiO$_2$. The titanium oxide slurry was warmed to 85°C, to which a 10 mass% aqueous solution of polyaluminum chloride (PAC: $[Al_3(OH)_nCl_{6-n}]_m$) was added and then aged for 10 minutes. The amount of the PAC added was 6 parts by mass as Al$_2$O$_3$ based on 100 parts by mass of the titanium oxide powder with a specific surface area of approximately 80 m$^2$/g or 4 parts by mass as Al$_2$O$_3$ based on 100 parts by mass of the titanium oxide powder with a specific surface area of approximately 60 m$^2$/g. The pH was adjusted to 6.0 using a 48 mass% aqueous solution of sodium hydroxide, followed by aging for 30 minutes and addition of sodium stearate. The amount of sodium stearate added was 7 parts by mass based on 100 parts by mass of the titanium oxide powder with a specific surface area of approximately 80 m$^2$/g or 5 parts by mass based on 100 parts by mass of the titanium oxide powder with a specific surface area of approximately 60 m$^2$/g. After adding sodium stearate and aging for 60 minutes, the pH was adjusted to 6.0 with 50 mass% sulfuric acid, followed by aging for another 30 minutes to obtain a slurry, which was filtered and washed with water to provide a cake of titanium oxide coated with a layer of aluminum hydroxide and stearic acid (or aluminum stearate). The cake was dried at 110°C, then pulverized by the impact pulverizer, and then sieved through a 0.3 mm sieve to provide a surface-coated titanium oxide powder. Titanium oxide particles in the powder thus obtained were coated with a layer of aluminum hydroxide and stearic acid (or aluminum stearate). The particles contained in the titanium oxide powder thus surface treated had an average short-axis length and an average aspect ratio almost the same as those of the titanium oxide powder before the surface treatment.

The surface-coated titanium oxide powders thus obtained with a specific surface area of approximately 80 m$^2$/g and a specific surface area of approximately 60 m$^2$/g were used to prepare emulsified preparations with a titanium oxide concentration of 25 mass%. In addition, the surface-coated titanium oxide powder with a specific surface area of approximately 60 m$^2$/g was used to prepare an emulsified preparation with a titanium oxide concentration of 10 mass%. All the emulsified preparations were expected to be cosmetics.

Emulsified preparation with concentration of 25 mass%

In a 100 mL bottle (J bottle, round shape wide mouth, manufactured by Nikko Hansen & Co., Ltd.), 30.87 g of a mixture of oil phase materials listed below, 22.05 g of the surface-coated titanium oxide powder with a specific surface area of approximately 80 m$^2$/g, and 130 g of φ 0.5 mm zirconia beads (produced by Shinmaru Enterprises Corp.) as a dispersion medium were put, and dispersed in a paint conditioner (1400-OH produced by RED DEVIL) for 5 hours, followed by separating the zirconia beads to prepare an oil phase dispersion. In a 100 mL polypropylene cup, 37.80 g of the oil phase dispersion was put and the dispersion was stirred at a stirring speed of 3000 rpm using a high-speed emulsifying/dispersing machine ("T.K. Robomix" manufactured by Primix Corp.) while 25.2 g of a mixture of aqueous phase materials listed below was added and the mixture was stirred continuously at 3000 rpm for 5 minutes to prepare an emulsified preparation.

(Oil phase materials)

- 21.07 g of Cyclopentasiloxane: "KF-995" produced by Shin-Etsu Chemical Co., Ltd.
- 4.9 g of Liquid paraffin: "Moresco White P-70" produced by MORESCO Co., Ltd.
- 4.9 g of PEG-9-Dimethicone: "KF-6019" produced by Shin-Etsu Chemical Co., Ltd.

(Aqueous phase materials)

- 17.53 g of Ion-exchanged water
- 7.67 g of 1,3-Butylene glycol

The emulsified preparation thus obtained with a concentration of 25 mass% was applied on a PET film (Lumirror 100T60 produced by Panac Co., Ltd.) using an automatic bar coater with an attached wire bar (No. 6) and dried by standing at room temperature for 24 hours to form a coating film. The color difference ΔE of the coating film thus formed from the Bioskin was evaluated in accordance with the above method. As a result, the ΔE was 4.8. In addition, an emulsified preparation with a concentration of 25 mass% was prepared and evaluated in the same manner using the surface-coated titanium oxide powder with a specific surface area of approximately 60 $m^2/g$, and the ΔE was 6.4.

Emulsified preparation with concentration of 10 mass%

In a 100 mL bottle (J bottle, round shape wide mouth, manufactured by Nikko Hansen & Co., Ltd.), 38.50 g of a mixture of oil phase materials listed below, 8.75 g of the surface-coated titanium oxide powder with a specific surface area of approximately 60 $m^2/g$, and 130 g of φ 0.5 mm zirconia beads (produced by Shinmaru Enterprises Corp.) as a dispersion medium were put, and dispersed in a paint conditioner (1400-OH produced by RED DEVIL) for 5 hours, followed by separating the zirconia beads to prepare an oil phase dispersion. In a 100 mL polypropylene cup, 37.80 g of the oil phase dispersion was put and the dispersion was stirred at a stirring speed of 3000 rpm using a high-speed emulsifying/dispersing machine ("T.K. Robomix" manufactured by Primix Corp.) while 32.2 g of a mixture of aqueous phase materials listed below was added and the mixture was stirred continuously at 3000 rpm for 5 minutes to prepare an emulsified preparation. The viscosity of the emulsified preparation was measured under the conditions of a No. 2 rotor, 25°C, and 60 rpm using a Brookfield viscometer, and the viscosity was 72 mPa•s.

(Oil phase materials)

- 29.75 g of Dimethicone: "KF-96L-1.5cs" produced by Shin-Etsu Chemical Co., Ltd.
- 4.375 g of Liquid paraffin: "Moresco White P-70" produced by MORESCO Co., Ltd.
- 4.375 g of PEG-9-Polydimethylsiloxyethyl dimethicone: "KF-6028P" produced by Shin-Etsu Chemical Co., Ltd.

(Aqueous phase materials)

- 22.4 g of Ion-exchanged water
- 9.8 g of 1,3-Butylene glycol

The emulsified preparation thus obtained with a concentration of 10 mass% was applied on a PET film (Lumirror 100T60 produced by Panac Co., Ltd.) using an automatic bar coater with an attached wire bar (No. 6) to form a coating film. The color difference ΔE of this coating film from the Bioskin, immediately after application, was evaluated in accordance with the above method to have a result of the ΔE of 6.4, whereas the color difference ΔE after drying by standing at room temperature for 24 hours was 4.0. In this situation, the color difference ΔE immediately after applying an emulsified preparation of Comparative Example 3 using a commercially available titanium oxide powder was 6.4, which is the same as that of the emulsified preparation of the present Example, while the color difference ΔE after drying was 4.4, which is greater than that of the emulsified preparation of the present Example. It was thus understood that the emulsified preparation of the present Example had improved transparency after drying compared with products in the past.

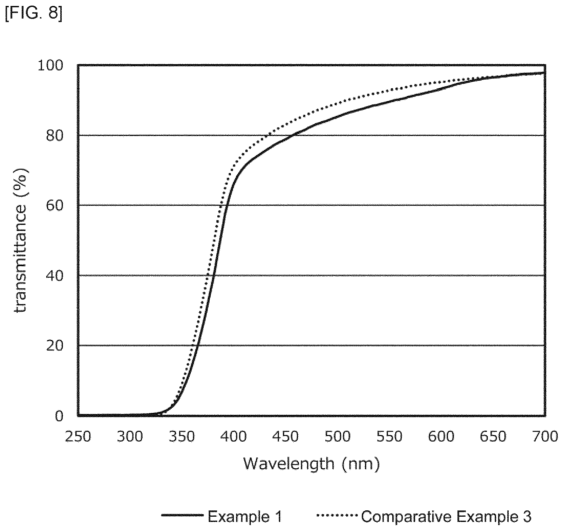

This emulsified preparation was applied on a polypropylene film (plain OPP sheet #40 produced by Mitsui Chemicals Tohcello, Inc.) using an automatic bar coater with an attached wire bar (No. 6) to form a coating film. This coating film was used to measure the light transmittance with a spectrophotometer ("U-4100" with use of an integrating sphere manufactured by Hitachi High-Technologies Corp.) under the following conditions. Fig. 8 illustrates a graph plotting the transmittance against the wavelength together with the transmittance of the emulsified preparation of Comparative Example 3. It was understood that the UVA (from 320 to 400 nm) shielding effect is greater compared with that in Comparative Example 3 using the commercially available titanium oxide powder. The transmittance at 400 nm was 66.0%, the transmittance at 450 nm was 78.9%, and the wavelength at a transmittance of 50% was 374 nm.

- Scan speed: 300 nm/min
- Sampling interval: 2 nm
- Measurement wavelength: from 250 to 700 nm

In addition, the feeling of use, the whiteness, the bluish color, and the transparency of the emulsified preparations thus obtained were evaluated in accordance with the above methods for voting which one of the emulsified preparation of the present Example or the emulsified preparation of Comparative Example 3 using the commercially available titanium oxide powder. As a result, the emulsified preparation of the present Example had lower viscosity compared with the emulsified preparation of Comparative Example 3, was more readily spread immediately after being dropped on the skin, and had substantially reduced unsmooth feeling typical of the powder, and thus all the 10 panelists determined that the emulsified preparation of the present Example had good feeling of use. Regarding the whiteness, the bluish color, and the transparency after spreading and drying as well, all the 10 panelists determined that the emulsified preparation using the titanium oxide powder of the present Example had more suppressed whiteness and bluish color and exhibited better transparency.

Moreover, in order to cause the emulsified preparation to have a viscosity closer to the viscosity of the emulsified preparation of Comparative Example 3, an emulsified preparation with a concentration of 10 mass% was prepared in the same manner as above except for changing the mixture of oil phase materials as listed below. The viscosity after adjustment was measured under the conditions of a No. 3 rotor, 25°C, and 60 rpm using a Brookfield viscometer, and the viscosity was 1278 mPa·s.

(Oil phase materials)

- 28.33 g of Dimethicone: "KF-96L-1.5cs" produced by Shin-Etsu Chemical Co., Ltd.
- 4.375 g of Liquid paraffin: "Moresco White P-70" produced by MORESCO Co., Ltd.
- 4.375 g of PEG-9-Polydimethylsiloxyethyl dimethicone: "KF-6028P" produced by Shin-Etsu Chemical Co., Ltd.
- 1.42 g of Aluminum stearate

The emulsified preparation after the viscosity adjustment was uniformly applied on a PMMA plate in an amount of 1.3 mg/cm$^2$, followed by air drying at room temperature for 30 minutes to measure the SPF and UVAPF using a SPF analyzer (Labsphere UV-2000S from Optometrics). As a result, the SPF was 12.6 and it was understood that the UVB was effectively shielded. The UVAPF was 4.7 and it was understood that the UVA was also effectively shielded. The results of the above evaluation are summarized in Table 4.

Example 2

A titanium oxide powder was produced in the same manner as that in Example 1 except for aging the slurry after adding hydrochloric acid in a range from 100°C to 105°C for 30 minutes for analysis and evaluation. The results of evaluation are summarized in Tables 1 through 3.

Example 3

A titanium oxide powder was produced in the same manner as that in Example 1 except for aging the slurry after adding hydrochloric acid at 50°C for 20 minutes for analysis and evaluation. The results of evaluation are summarized in Tables 1 through 3. For the titanium oxide powder (after calcination) with a specific surface area of approximately 60 m$^2$/g, only the specific surface area was measured.

Comparative Example 1

A titanium oxide powder was produced in the same manner as that in Example 1 except for adding 10.0 kg of 35 mass% hydrochloric acid to the slurry containing sodium titanate and heating for aging in a range from 100°C to 105°C for 5 hours for analysis and evaluation. The results of evaluation are summarized in Tables 1 through 3.

Comparative Example 2

This is an example of not impregnating with a nitrogenous compound. To the slurry containing sodium titanate, 14.0 kg of 35 mass% hydrochloric acid was added and heated for aging at 80°C for 10 minutes. The slurry after aging was diluted with water to obtain a slurry containing titanium oxide of 70 g/L in terms of $TiO_2$. The titanium oxide thus obtained contained rutile-type crystals. The titanium oxide slurry was warmed to 80°C and adjusted to pH 6.4 with a 24 mass% aqueous sodium hydroxide solution, followed by aging for 30 minutes. After the aging was completed, the slurry readjusted to pH 6.4 with a 24 mass% aqueous sodium hydroxide solution or hydrochloric acid was filtered and washed to obtain a cake of titanium oxide. This cake was dried, pulverized, and sieved in the same manner as in Example 1 to obtain a titanium oxide powder (before calcination). In the same manner as in Example 1 in the later procedure, a titanium oxide powder (after calcination) was produced for analysis and evaluation. The results of evaluation are summarized in Tables 1 through 3.

Example 4

This is an example of spraying aqueous ammonia immediately before calcination. A titanium oxide powder (after calcination) was produced for analysis and evaluation in the same manner as in Comparative Example 2 except for spraying 10 parts by mass of 24 mass% aqueous ammonia based on 100 parts by mass of a titanium oxide powder (before calcination) after drying obtained in the same manner as in Comparative Example 2. The results of evaluation are summarized in Tables 1 through 3.

Comparative Example 3

An emulsified preparation was prepared under the same conditions, in the emulsified preparation with a concentration of 10 mass% produced in Example 1, except for using a titanium oxide powder a commercially available titanium oxide powder ("MT-100Z" produced by Tayca Corp.) instead of the surface coated titanium oxide powder and changing the dispersion time in the paint conditioner to 2 hours. The "MT-100Z" is a titanium oxide powder containing spindle shaped particles, has an average short-axis length of 7.9 nm and an average aspect ratio of 4.0, and is surface treated with polyaluminum chloride and sodium stearate in the same manner as Example 1. Fig. 4 illustrates an ESCA spectrometry chart for this powder. It was understood that no peaks were observed from 395 to 402 eV. The reasons for reducing the dispersion time in this example are because the viscosity increased during the dispersion and the color difference ΔE of the coating film immediately after application from the Bioskin became equivalent (6.4) to the product of the present invention. The viscosity of the emulsified preparation was measured under the conditions of a No. 3 rotor, 25°C, and 60 rpm using a Brookfield viscometer, and the viscosity was 1492 mPa•s. The results of evaluating the emulsified preparation thus obtained in the same manner as in Example 1 are summarized in Table 4.

[Table 1]

| | Aging Conditions | | Neutralization Method | Specific Surface Area | TEM Observation | | | Color Difference | | |
|---|---|---|---|---|---|---|---|---|---|---|
| | Temperature | Time | | | Long-Axis Length | Short-Axis Length | Aspect Ratio | L* | a* | b* |
| | (°C) | (min) | | (m²/g) | (nm) | (Cm) | | | | |
| Example 1 | 80 | 10 | NH$_3$ | 204 | 21 | 6 | 3.5 | 97.99 | -0.53 | 2.41 |
| Example 2 | 100-105 | 30 | NH$_3$ | 152 | 50 | 10 | 5.0 | 98.02 | -0.54 | 2.21 |
| Example 3 | 50 | 20 | NH$_3$ | 242 | *1) | *1) | *1) | 96.96 | -0.45 | 2.85 |
| Example 4 | 80 | 10 | NaOH | 197 | 21 | 6 | 3.5 | 97.61 | -0.28 | 1.78 |
| Comparative Example 1 | 100-105 | 300 | NH$_3$ | 113 | 60 | 13 | 4.6 | 98.08 | -0.27 | 1.50 |
| Comparative Example 2 | 80 | 10 | NaOH | 197 | 21 | 6 | 3.5 | 97.61 | -0.28 | 1.78 |
| *1) Particles were too small to perform image processing. | | | | | | | | | | |

[Table 2]

| | Calcination Temperature | Specific Surface Area | TEM Observation | | | ESCA | | Color Difference | | | Absorbance | | | | NC Coating Film | Emulsion Coating Film |
| | | | Long-Axis Length | Short-Axis Length | Aspect Ratio | 397 eV | 400 eV | L* | a* | b* | 320 nm | 450 nm | 600 nm | 450 nm/ 320 nm | ΔE | ΔE |
| | (°C) | (m²/g) | (nm) | (nm) | | | | | | | | | | | | |
| Example 1 | 380 | 82.0 | 20.3 | 12.7 | 1.60 | Found | Found | 96.04 | -1.66 | 7.93 | 1.041 | 0.088 | 0.014 | 0.085 | 9.6 | 4.8 |
| Example 2 | 300 | 78.4 | 28.7 | 14.8 | 1.94 | Found | Found | 95.65 | -1.19 | 6.75 | 1.040 | 0.070 | 0.024 | 0.067 | 9.7 | 5.5 |
| Example 3 | 400 | 83.5 | 22.4 | 14.1 | 1.59 | Found | Found | 92.82 | -2.90 | 22.46 | 0.967 | 0.251 | 0.033 | 0.260 | 8.7 | 4.9 |
| Example 4 | 380 *1) | 80.5 | 20.6 | 12.3 | 1.67 | Found | Found | 95.76 | -1.94 | 9.82 | 1.055 | 0.084 | 0.014 | 0.080 | 9.3 | 4.6 |
| Comparative Example 1 | 220 | 81.5 | 36.7 | 15.8 | 2.32 | Not Found | Found | 96.82 | -0.12 | 2.05 | 1.003 | 0.011 | 0.008 | 0.011 | 11.4 | 7.3 |
| Comparative Example 2 | 430 | 79.6 | 22.9 | 13.5 | 1.70 | Not Found | Found | 97.65 | -0.31 | 2.45 | 1.060 | 0.004 | 0.003 | 0.004 | 10.1 | 5.6 |

*1) 10 mass% of 24 mass% aqueous ammonia was added for calcination.

EP 4 342 848 A1

[Table 3]

| | Calcination Temperature | Specific Surface Area | TEM Observation | | | ESCA | | Color Difference | | | Absorbance | | | | NC Coating Film | Emulsion Coating Film |
|---|---|---|---|---|---|---|---|---|---|---|---|---|---|---|---|---|
| | | | Long-Axis Length | Short-Axis Length | Aspect Ratio | 397 eV | 400 eV | L* | a* | b* | 320 nm | 450 nm | 600 nm | 450 nm/ 320 nm | ΔE | ΔE |
| | (°C) | (m²/g) | (nm) | (nm) | | | | | | | | | | | | |
| Example 1 | 480 | 60.3 | 34.6 | 22.4 | 1.54 | Not Found | Found | 96.58 | -1.37 | 7.58 | 1.034 | 0.081 | 0.007 | 0.078 | 10.2 | 6.4 |
| Example 2 | 410 | 62.0 | 31.8 | 17.9 | 1.78 | Not Found | Found | 96.36 | -1.23 | 7.64 | 1.012 | 0.069 | 0.011 | 0.068 | 10.3 | 6.8 |
| Example 3 | 510 | 58.9 | - | - | - | - | - | - | - | - | - | - | - | - | - | - |
| Example 4 | 480 *1) | 62.4 | 31.3 | 18.7 | 1.67 | Not Found | Found | 94.34 | -1.74 | 10.08 | 1.027 | 0.105 | 0.014 | 0.102 | 9.8 | 6.0 |
| Comparative Example 1 | 350 | 60.4 | 35.9 | 20.0 | 1.80 | Not Found | Found | 97.52 | -0.44 | 2.76 | 1.006 | 0.011 | 0.001 | 0.011 | 12.5 | 8.4 |
| Comparative Example 2 | 530 | 63.1 | 33.6 | 20.3 | 1.66 | Not Found | Found | 97.71 | -0.27 | 2.61 | 1.061 | 0.006 | 0.003 | 0.006 | 11.2 | 7.0 |

*1) 10 mass% of 24 mass% aqueous ammonia was added for calcination.

[Table 4]

| | Viscosity | Transmittance | | | Emulsion Coating Film | | Sensory Evaluation | | | | SPF | UVAPF |
|---|---|---|---|---|---|---|---|---|---|---|---|---|
| | | 400 nm | 450 nm | 50% Transmittance | Immediately After Application | After Drying | Feeling of Use | Whiteness | Bluish Color | Transparency | | |
| | (mPa•s) | (%) | (%) | (nm) | $\Delta E$ | $\Delta E$ | (person) | (person) | (person) | (person) | | |
| Example 1 | 72 | 66.0 | 78.9 | 374 | 6.4 | 4.0 | 10 | 10 | 10 | 10 | 12.6 | 4.7 |
| Comparative Example 3 | 1492 | 71.1 | 83.0 | 368 | 6.4 | 4.4 | 0 | 0 | 0 | 0 | 9.3 | 3.3 |

**Claims**

1. A titanium oxide powder, wherein a ratio ($I_A/I_R$) of a peak intensity ($I_A$) of anatase-type crystals to a peak intensity ($I_R$) of rutile-type crystals as measured by X-ray diffractometry is 0.1 or less,

   particles contained in the powder have an average short-axis length from 10 to 50 nm and an average aspect ratio from 1 to 3,
   a ratio ($A_{450}/A_{320}$) of an absorbance $A_{450}$ at a wavelength of 450 nm to an absorbance $A_{320}$ at a wavelength of 320 nm is from 0.015 to 0.5, and
   the powder contains a nitrogen atom and a peak derived from the nitrogen atom is observed from 395 to 402 eV in ESCA (Electron Spectroscopy for Chemical Analysis) measurement.

2. The titanium oxide powder according to Claim 1, wherein no peaks of anatase-type crystals are observed in the X-ray diffractometry.

3. The titanium oxide powder according to Claim 1 or 2, wherein the powder has a specific surface area from 25 to 100 $m^2$/g.

4. The titanium oxide powder according to any one of Claims 1 through 3, wherein an absorbance $A_{600}$ at a wavelength of 600 nm is 0.1 or less.

5. The titanium oxide powder according to any one of Claims 1 through 4, wherein in an L*a*b* color scale, an L* value is from 92 to 99, an a* value is from -5 to 2, and a b* value is from 3 to 30.

6. The titanium oxide powder according to any one of Claims 1 through 4, wherein the surface of the particles contained in the powder is coated with an inorganic compound and/or organic compound layer.

7. The titanium oxide powder according to Claim 6, wherein the surface of the particles is coated with an inorganic compound layer and the inorganic compound contains at least one element selected from the group consisting of aluminum, magnesium, calcium, silicon, zinc, titanium, zirconium, iron, cerium, and tin.

8. The titanium oxide powder according to Claim 6 or 7, wherein the surface of the particles is coated with an organic compound layer and the organic compound is at least one selected from the group consisting of a fatty acid or a salt thereof, a silicone compound, a coupling agent, and a fluorine compound.

9. The titanium oxide powder according to any one of Claims 6 through 8, wherein in an L*a*b* color scale, an L* value is from 92 to 99, an a* value is from -5 to 2, and a b* value is from 2 to 30.

10. A dispersion comprising the titanium oxide powder according to any one of Claims 1 through 9 dispersed in a dispersion medium.

11. A cosmetic comprising the titanium oxide powder according to any one of Claims 1 through 9.

12. A paint comprising the titanium oxide powder according to any one of Claims 1 through 9.

13. An ink comprising the titanium oxide powder according to any one of Claims 1 through 9.

14. A toner comprising, as an external additive, the titanium oxide powder according to any one of Claims 1 through 9.

15. A method for producing a titanium oxide powder, comprising:

    an alkalization step of adding an alkali metal hydroxide to an aqueous dispersion of hydrous titanium oxide to provide an alkali metal titanate;
    an acidification step of adding hydrochloric acid to the aqueous dispersion of the alkali metal titanate to provide titanium oxide containing rutile-type crystals;
    an impregnation step of impregnating the titanium oxide with a nitrogenous compound; and
    a calcination step of calcining from 200 to 600°C.

16. The method for producing a titanium oxide powder according to Claim 15, wherein the titanium oxide before calcining has a specific surface area from 120 to 300 m$^2$/g.

17. The method for producing a titanium oxide powder according to Claim 15 or 16, wherein in the impregnation step, the hydrochloric acid remained after the acidification step is neutralized by a basic nitrogenous compound and the titanium oxide is impregnated with the nitrogenous compound.

[FIG. 1]

[FIG. 2]

[FIG. 3]

[FIG. 4]

[FIG. 5]

[FIG. 6]

[FIG. 7]

[FIG. 8]

## INTERNATIONAL SEARCH REPORT

| International application No. |
| --- |
| **PCT/JP2022/020415** |

**A. CLASSIFICATION OF SUBJECT MATTER**

*C01G 23/053*(2006.01)i; *C09C 1/36*(2006.01)i; *C09C 3/06*(2006.01)i; *C09C 3/08*(2006.01)i; *A61Q 17/04*(2006.01)i; *C09D 201/00*(2006.01)i; *C09D 11/00*(2014.01)i; *A61K 8/29*(2006.01)i; *C09D 7/62*(2018.01)i
FI: C01G23/053; C09C1/36; C09C3/06; C09C3/08; C09D7/62; C09D201/00; C09D11/00; A61K8/29; A61Q17/04

According to International Patent Classification (IPC) or to both national classification and IPC

**B. FIELDS SEARCHED**

Minimum documentation searched (classification system followed by classification symbols)

C01G23/053; C09C1/36; C09C3/06; C09C3/08; A61Q17/04; C09D201/00; C09D11/00; A61K8/29; C09D7/62

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2014-084251 A (TITAN KOGYO KK) 12 May 2014 (2014-05-12)<br>claims, examples | 1-17 |
| A | JP 2010-173863 A (TITAN KOGYO KK) 12 August 2010 (2010-08-12)<br>claims, examples | 1-17 |
| A | CN 105858722 A (CHINA TOBACCO FUJIAN INDUSTRIAL CO., LTD.) 17 August 2016<br>(2016-08-17)<br>claims | 1-17 |

☐ Further documents are listed in the continuation of Box C.　　☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **17 June 2022** | **05 July 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2022/020415**

| Patent document cited in search report | | | Publication date (day/month/year) | Patent family member(s) | | | Publication date (day/month/year) |
|---|---|---|---|---|---|---|---|
| JP | 2014-084251 | A | 12 May 2014 | US<br>claims, examples<br>EP | 2014/0112965<br><br>2724985 | A1<br><br>A1 | |
| JP | 2010-173863 | A | 12 August 2010 | US<br>claims, examples<br>EP | 2010/0189666<br><br>2216296 | A1<br><br>A2 | |
| CN | 105858722 | A | 17 August 2016 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010173863 A
- JP 2011001199 A
- JP 2014084251 A
- WO 2020230812 A

**Non-patent literature cited in the description**

- **J. LIU et al.** *Catalysts,* 2020, vol. 10, 1126